# EUROPEAN PATENT SPECIFICATION

(11) **EP 4 213 696 B1**
(45) Date of publication and mention of the grant of the patent: **22.04.2026**
(21) Application number: 21786551.8
(22) Date of filing: 20.09.2021
(51) Int. Cl.: A61B 1/00, A61B 1/04, F16L 55/32

(54) **STEERABLE ENDOSCOPE**
LENKBARES ENDOSKOP
ENDOSCOPE ORIENTABLE

(30) Priority: 21.09.2020 GB 202014844
(43) Date of publication of application: 26.07.2023
(73) Proprietor: Majeed, Ali, Sheffield, South Yorkshire S10 3HJ (GB)
(72) Inventor: Majeed, Ali, Sheffield, South Yorkshire S10 3HJ (GB)
(74) Representative: HGF
(86) International application number: PCT/GB2021/052433
(87) International publication number: WO 2022/058751

(56) References cited:
- WO-A1-2011/040137
- DE-B3- 102011 017 343
- US-A1- 2002 156 347
- US-A1- 2007 185 381
- US-A1- 2010 001 592
- FORMOSA GREGORY A ET AL: "Novel Optimization-Based Design and Surgical Evaluation of a Treaded Robotic Capsule Colonoscope", IEEE TRANSACTIONS ON ROBOTICS, IEEE SERVICE CENTER, PISCATAWAY, NJ, US, vol. 36, no. 2, 1 April 2020 (2020-04-01), pages 545 - 552, XP011781862, ISSN: 1552-3098, [retrieved on 20200402], DOI: 10.1109/TRO.2019.2949466

## Description

This invention relates to the field of endoscopy, in particular but not exclusively to the field of self-propelled steerable capsule endoscopes for use in the endoscopic examination of the gastrointestinal tract or other hollow organs of the body.

### BACKGROUND

Flexible colonoscopy is the current gold standard for visual inspection of the colon lining. The procedure is uncomfortable, often requiring heavy sedation and analgesia, and has a small but significant risk of colonic perforation (which may require emergency surgery and formation of a colostomy). Capsule endoscopy, in which the patient swallows a capsule containing a small camera, is much more comfortable however such capsule endoscopes are passive devices which cannot be steered or controlled and whose camera view depends entirely on which way the camera is facing when the image is captured.

A number of attempts have been made to develop a steerable endoscope but these have not met with success due to the difficulty in developing a safe and reliable propulsion mechanism which does not damage the mucosa of the bowel. The mucosa is the moist inner lining of body cavities and internal tracts (for example the gastrointestinal tract)

Known prior art devices include:
External magnets used to control the movement of a capsule endoscope, as referred to in World J Gastroenterol 2013 January 28; 19(4): 431-439. This is new and experimental technology but many major manufacturers of capsule endoscopes are moving in the direction of developing magnetically controlled systems.

The Endotics^{™} system from Era Endoscopy s.r.l. and that described in Int J Med Robot. 2013 Sep;9(3):371-8 are semi-autonomous devices capable of propelling themselves along a flexible environment in the manner of an inchworm, e.g. using suction cups attachable to the mucosa to pull the device. These devices are slow-moving and their orientation cannot be readily controlled.

A micro creeping robot based on the earthworm is described in J Med Eng Technol. 2005 Jan-Feb;29(1):1-7 . This is a device in which a micro-robot creeps in declining rubber tubes. This device is slow-moving and not steerable.

Several devices having movable legs have been considered. https://www.telegraph.co.uk/news/health/news/6300636/Spider-pill-offers-new-way-to-scan-for-diseases-including-colon-cancer.html describes a swallowable pill which contains a tiny camera and is fitted with tiny legs that can be activated remotely once it is inside the colon or intestine. The legs protrude outwards and are movable in order to make device to 'crawl' inside the patient like a spider. It can be moved back and forth, giving doctors more flexibility during the examination. Another spider-like device is described in Gastrointestinal Endoscopy Volume 67, Issue 7, Pages 1153-1158, June 2008. Devices with legs are generally slow and not steerable and, in particular, the legs of the devices risk trauma to the mucosa and underlying tissue, as well as difficulties with traction when in contact with the mucosa.

https://www.popsci.com/science/article/2011-06/worlds-first-self-propelled-endoscopy-device-swims-entire-digestive-tract-mere-hours/ describes a device designed to swim like a tadpole with a paddle at the back thereof. This device must be fully submerged in liquid and is not steerable.

Document US 2002/156347 A1 discloses a self-propelled capsule endoscope with at least two propellers having blades to drive and steer the capsule by tissue contact.

It is an object of the present invention to provide a steerable endoscope that mitigates some of the above-mentioned disadvantages.

### BRIEF SUMMARY OF THE DISCLOSURE

In accordance with the present invention there is provided a steerable endoscope as defined in claim 1 and comprising:
a capsule body having a longitudinal axis and having at least one axle channel extending transversely therein;
at least two propellers, each located on an axle located in said at least one axle channel, each propeller comprising a plurality of blades rotatable about a hub;
a camera and light source housed within said capsule body;
a drive mechanism, including a differential steering mechanism, housed within said capsule body, the drive mechanism being capable of driving said propellers to rotate said blades into contact with the mucosa of an internal body cavity or tract such that the endoscope is capable of self-propelled steerable travel within the internal body cavity or tract.

The endoscope may be used in any suitable internal body cavity or tract, for example the gastrointestinal tract and is particularly but not exclusively for use in the colon.

Differential steering of the propellers permits greater control over steering than is possible with any of the known prior art devices. The disadvantages of a device which "swims" i.e. needs to be fully submerged in liquid are mitigated by the device of the invention whose blades are driven into contact with the mucosa in order to effect travel. Furthermore, the blades are much less likely to cause trauma to the mucosa than the conventional "legs" of prior art devices which crawl or walk.

In accordance with the present invention the blades comprise a flexible material and wherein the flexibility of the blades increases with distance from the hub. This further reduces risk of trauma to the mucosa, by having a relatively stiff region of the blade near to the hub to facilitate driving of the propeller (described below) combined with a very soft and flexible, feather-like region of the blade at its distal end where it will contact the tissue. The blades preferably comprise a resilient material.

To increase the surface area of the blade in contact with the mucosa and to further reduce the risk of trauma, the end of each blade distant from the hub can be divided into two or more tines.

In an embodiment, the propellers further comprise a strengthening brace, for example an annular strengthening brace, attached to each blade at a point intermediate the hub and a distal end of the blade.

In an embodiment, one or more of the blades comprises a surface feature to increase the surface area thereof, wherein the surface feature comprises an enlarged distal end, bumps, ridges, protrusions, and/or hair-like protrusions.

In some embodiments, the blades are symmetrical about a longitudinal plane passing through the centre of the hub. In other embodiments, the blades are not symmetrical about a longitudinal plane passing through the centre of the hub.

Preferably, the propellers are collapsible against the capsule body to temporarily reduce the outside dimensions of the endoscope. This facilitates insertion of the endoscope into the rectum or other internal body cavity or tract.

In an embodiment, the capsule body is sealed with respect to the or each axle.

In an embodiment, the drive mechanism comprises a conventional electric motor and a drive coupling including sealing rings to seal the axles from internal components.

In another embodiment, the steerable endoscope further comprises a magnetic drive coupling between the drive mechanism and the or each axle, the capsule body being wholly sealed from the or each axle. The magnetic drive coupling enables the capsule body and the components therein to be completely sealed from body fluids etc. Completely sealing the electronic components inside the capsule body in this way also means that the endoscope can be more easily sterilised, for example in an autoclave.

In an embodiment, the capsule body comprises an openable and closeable two-part body, having a substantially smooth external surface when closed. Preferably the closed capsule body is a spherocylinder or ovoid. The capsule body may have a substantially planar underside portion.

In an embodiment, said camera and light source comprises two cameras and light sources, one in each end of said capsule body.

The steerable endoscope may further comprise one or more connectors for releasable connection of the endoscope to an external power source and/or control cable and/or insufflation means.

Preferably, the drive mechanism comprises an independently-controllable motor for each propeller.

In an embodiment, the steerable endoscope comprises four of said propellers.

Preferably, said axle(s) do not protrude transversely beyond the external surface of the capsule body, or protrude only for a minimal distance.

In an embodiment, one of said axle channels on one side the capsule body is longitudinally offset from another of said axle channels on the other side of the capsule body.

In an embodiment, the steerable endoscope comprises two of said axle channels, offset from one another with respect to the longitudinal axis of the capsule body. Preferably, said offset axle channels are offset such that there is a clearance between one side of the outer surface of the capsule body and the distal end of the blades.

In an embodiment, the endoscope further comprises means for wiping or washing or other means for clearing the lens cover of the or each camera. The endoscope may be provided with a vibration motor so that the lens can be cleared by vibrating it against a mucosal surface.

In an embodiment, the endoscope further comprises a biopsy arm extendable from the capsule body.

### BRIEF DESCRIPTION OF THE DRAWINGS

Embodiments of the invention are further described hereinafter, by way of example only, with reference to the accompanying drawings, in which:
Figure 1 is a an exploded perspective view of an endoscope according to an aspect of the claimed invention;
Figure 2 is a schematic plan view of part of the endoscope showing two propellers;
Figure 3 is a schematic plan view of part of the endoscope showing the axles and axle channels;
Figure 4 is a schematic side view of part of the endoscope showing the external tether;
Figure 5 illustrates how a propeller blade could be divided into multiple tines;
Figure 6 is a schematic view of an embodiment of the propeller;
Figure 7 illustrates one blade having a textured surface;
Figure 8 is a schematic side view of part of the endoscope including a biopsy arm;
Figure 9 is a range of views of a propeller having a strengthening brace;
Figures 10 - 13 are each a range of views of further embodiments of the propeller;
Figure 14 is a range of views of an embodiment of a two-part capsule body;
Figure 15 is a range of views of another embodiment of the capsule body;
Figure 16 is a schematic side view of an embodiment of the endoscope having offset propellers; and
Figure 17 is a schematic side view of an embodiment of the endoscope having four propellers on each side of the capsule body.

### DETAILED DESCRIPTION

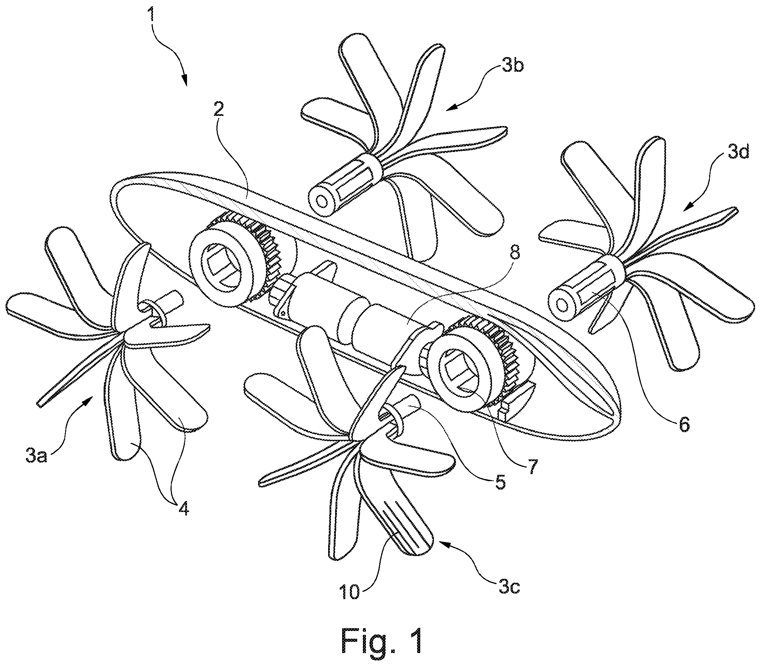

Referring to the Figures, in Figure 1, an example of an endoscope embodying the present invention is shown, partly in section. The endoscope 1 comprises a capsule body 2 having a generally smooth exterior surface in which components can be housed. The capsule body 2 may be spherocylindrical, ovoid or similar in shape and has a camera, lens 11 and light source 12 located in one or both of the narrowed ends of the capsule body.

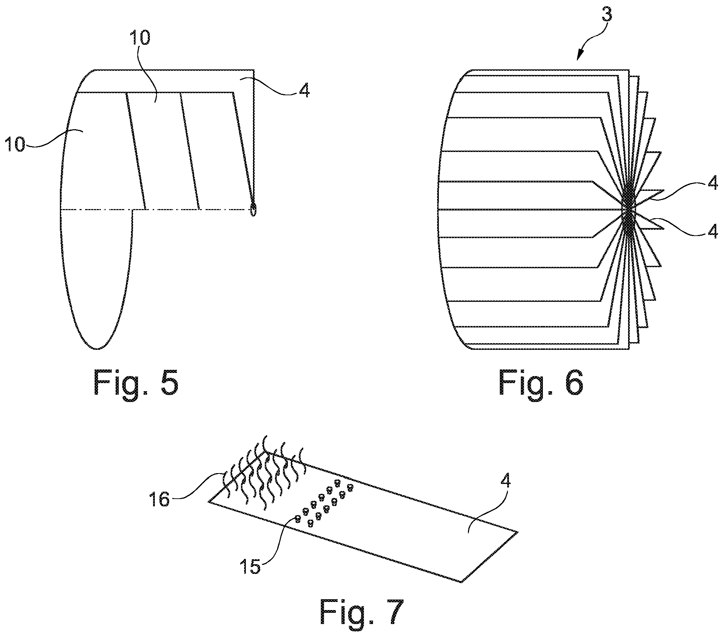

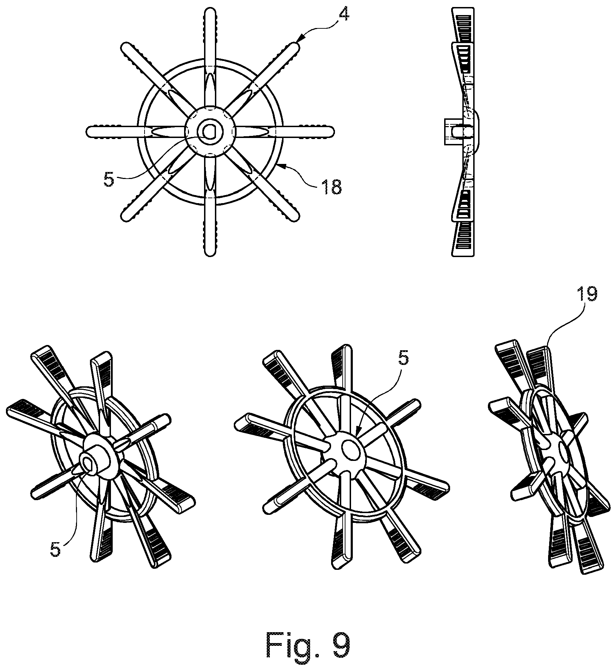

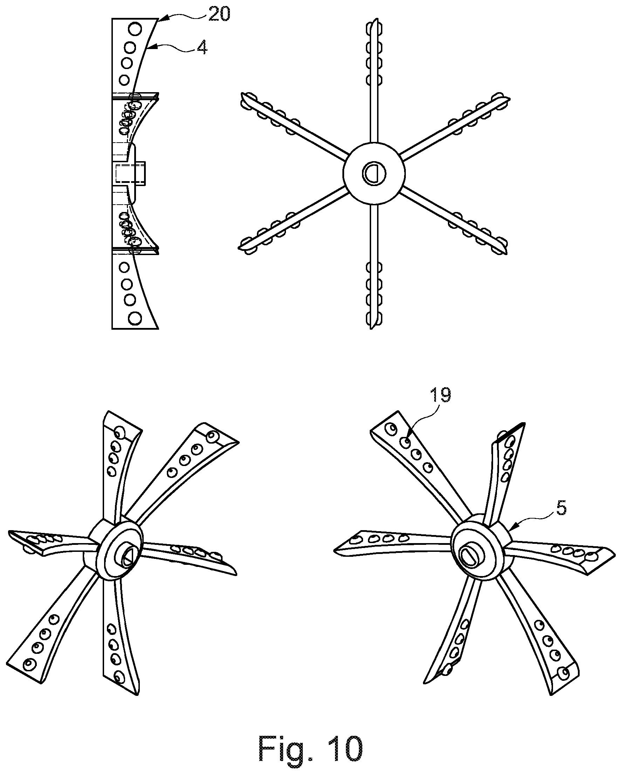

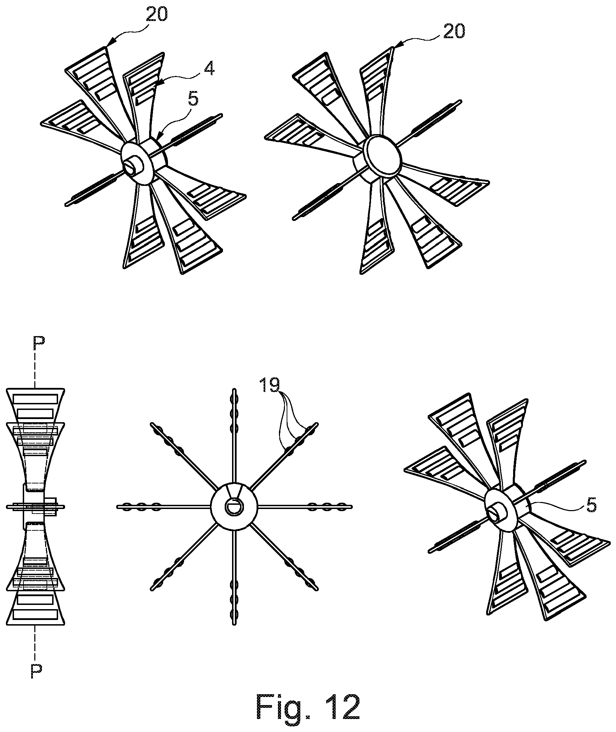

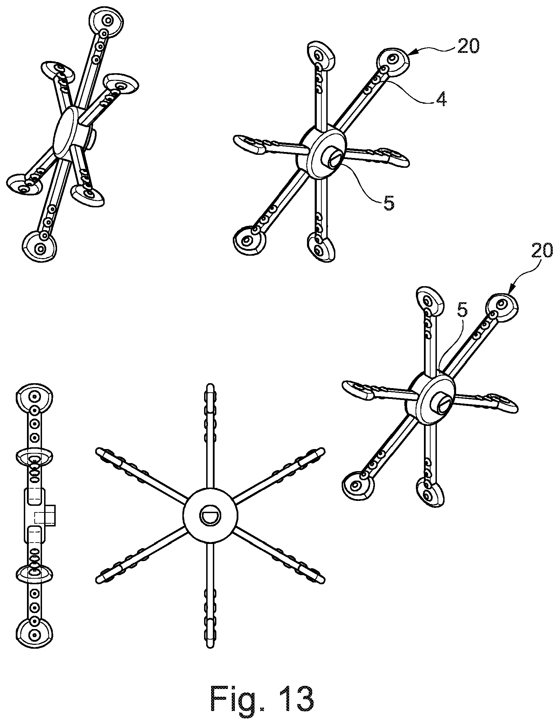

Four propellers 3a, 3b, 3c ,3d are provided, each of which comprises a plurality of blades 4 extending from a central hub 5 mounted on an axle 6. Each propeller may have the same number of blades or a different number. Each blade 4 may be unitary or may itself be divided into a plurality of tines 10 (see Figures 1 and 5). The blades may have a surface feature 19 to increase their surface area, for example a textured surface 15 or hair-like protrusions 16 as illustrated in Figure 7. The surface feature 19 may alternatively be a pattern of bumps, ridges, protrusions or the like, as in the embodiments illustrated in Figures 9 - 13. The distal end 20 of the blade 4 may be enlarged compared with the end of the blade nearest the hub 5 to further increase the surface area (shown particularly for example in the embodiments of Figures 12 and 13). The increased surface area resulting from the surface feature 19, optionally including the enlarged distal end 20, improves traction between the blades and the mucosa, reducing the likelihood of slippage.

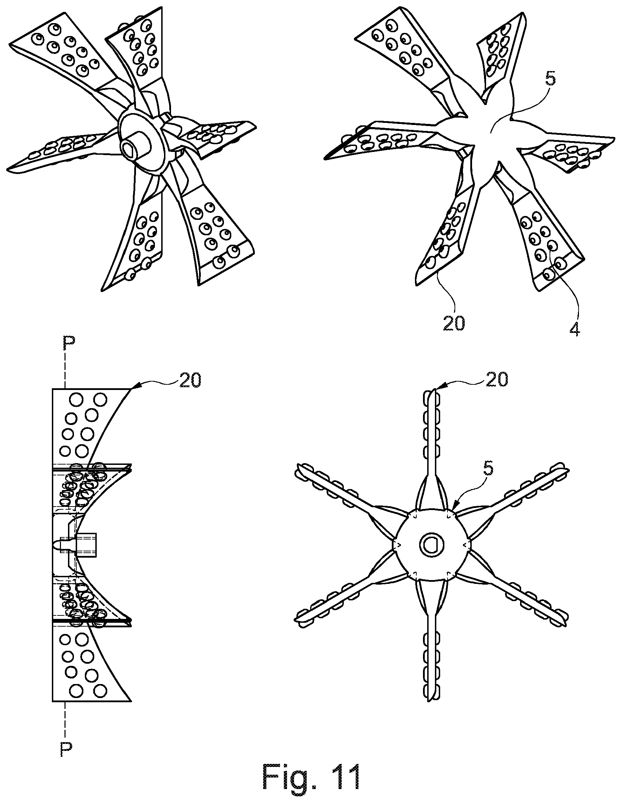

The blades 4 are made from a soft and flexible elastomer which is relatively stiff near the hub 5 but gradually becomes more flexible further away from the hub such that the distal end 20 of the blade (with respect to the hub) is extremely soft and highly unlikely to cause any damage to the mucosa. The distal end 20 of the blade may have a reduced thickness or taper, as shown in Figure 11 for example. Therefore the design of the blades is such that, at or near the hub 5, the blades are stiff enough to transmit torque provided by the rotating hub so that the blades rotate effectively, yet distal from the hub the blades are very soft and feather-like so as to minimise damage to the mucosa.

In some embodiments, for example that shown in Figure 11, the blades 4 are not symmetrical about a longitudinal plane P passing through the centre of the hub, the blades extending further in a direction away from the capsule.

In some embodiments, for example that shown in Figure 12, the blades 4 are symmetrical about a longitudinal plane P passing through the centre of the hub.

In the Figure 1 embodiment, the distal ends of the blades (furthest from the hub) are entirely separate and free from one another. In an alternative embodiment (not illustrated), the distal ends of the blades may be surrounded by a soft peripheral band or ring which helps keep the blades properly aligned. The width of the band is small compared with the width of the blade so that it does not significantly affect he blade's interaction with the mucosa. In another embodiment (an example of which is in Figure 9), the blades 4 are attached to an annular strengthening brace 18 intermediate their distal ends and the hub (similar to a ship's wheel). In an alternative embodiment, shown in Figure 11, instead of an annular strengthening brace, the hub 5 is enlarged in order to provide more support for the blades 4, with parts of the hub 5 extending into and merging with the blades such that the hub is generally star-shaped.

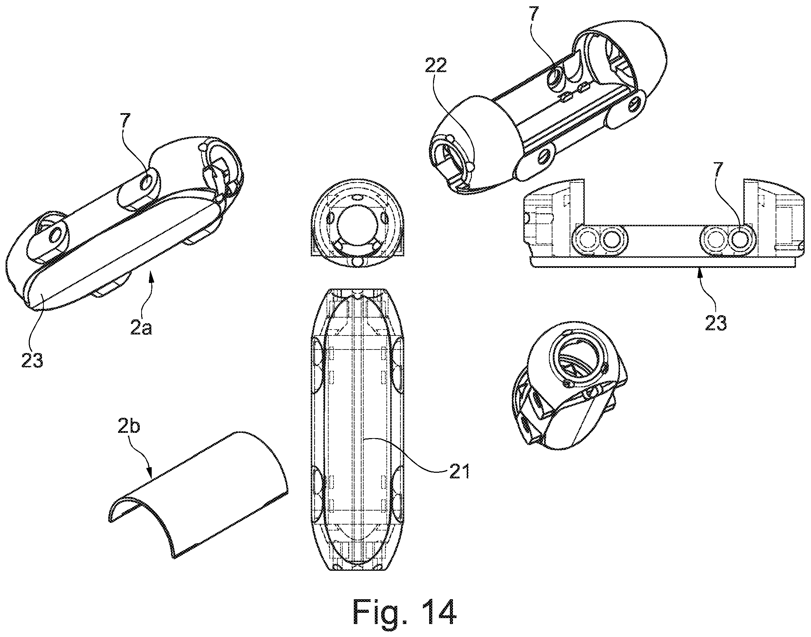

In the embodiment shown in Figure 14, the capsule body 2 is an openable and closeable two-part capsule body comprising a base 2a and a lid 2b which have a substantially smooth external surface when closed together enclosing and sealing the components inside. An underside portion 23 of the base 2a is substantially planar and includes a channel 21 which can house a tether comprising an external CO₂ or water delivery tube (not shown). Three equispaced LED windows 22 are located on one end of the capsule body.

The capsule body 2 is provided with axle channels 7 into which the axles can fit. The axles are sealed from the interior of the capsule by means of seals.

Referring to Figure 1, inside the axle channels 7, the axles 6 couple with a drive mechanism 8 housed inside the capsule body 2 which is capable of driving the axles 6 to rotate, rotating the hubs 5 and corresponding blades 4 of the propellers 3. A differential steering mechanism is used to independently control the respective speed and direction of rotation of each of the propellers 4.

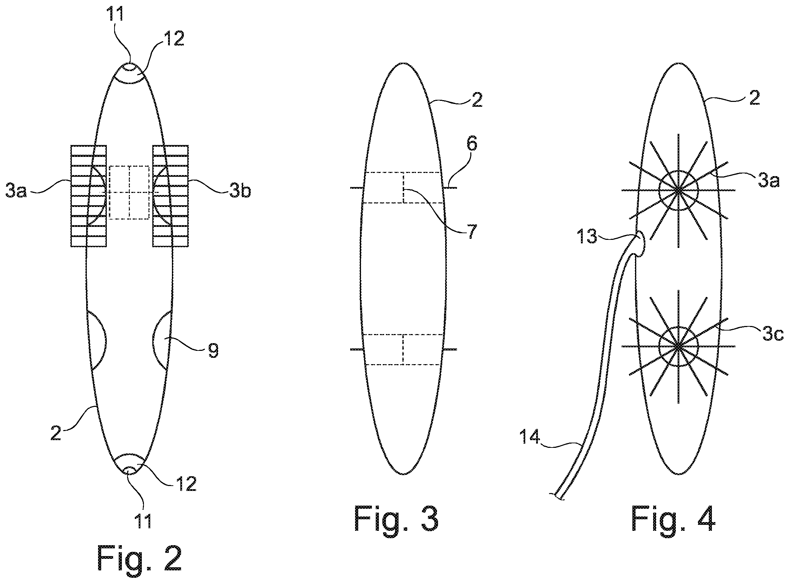

The drive mechanism 8 includes a separate motor for each of the propellers. The drive coupling may be located within the capsule body 2 so that neither the drive coupling nor the axles protrude from the exterior surface of the body, or only protrude by a very short amount. Recesses 9 may be provided in the exterior surface of the capsule body to facilitate this (see Figure 2).

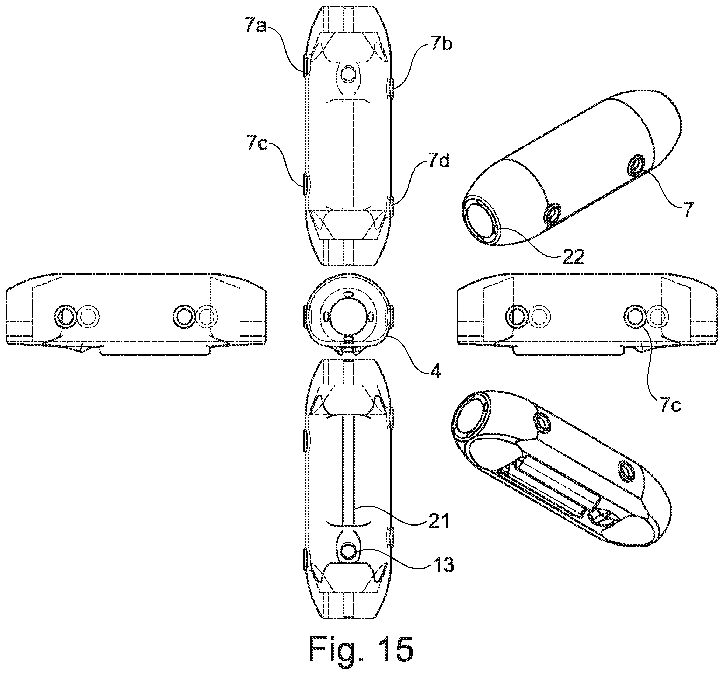

In the embodiment shown in Figure 15, the axle channels 7a, 7c on one side of the capsule body 2 are longitudinally offset from the axle channels 7b, 7d on the other side of the capsule body.

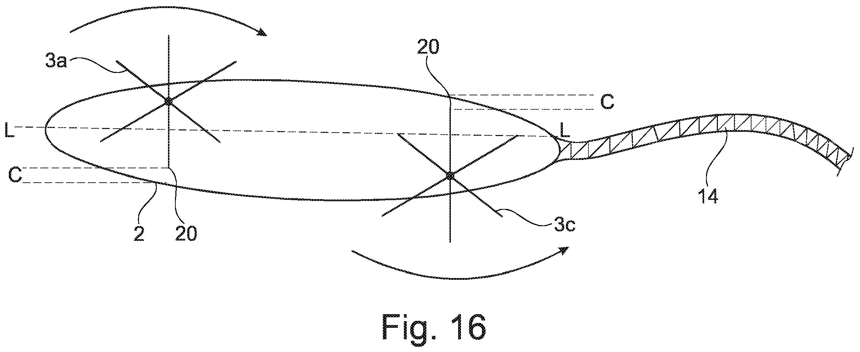

In the embodiment shown in Figure 16, the propellers 3a, 3c on the same side of the capsule body 2 are offset from each other with respect to the longitudinal axis L of the capsule body. This arrangement may be reproduced or mirrored with propellers 3b, 3d (not shown in Figure 16) and/or combined with the longitudinal offset shown in Figure 15. The offset either side of the longitudinal axis L means that, for each propeller, there is a clearance C between the distal end 20 of the blades and the external surface of the capsule body 2.

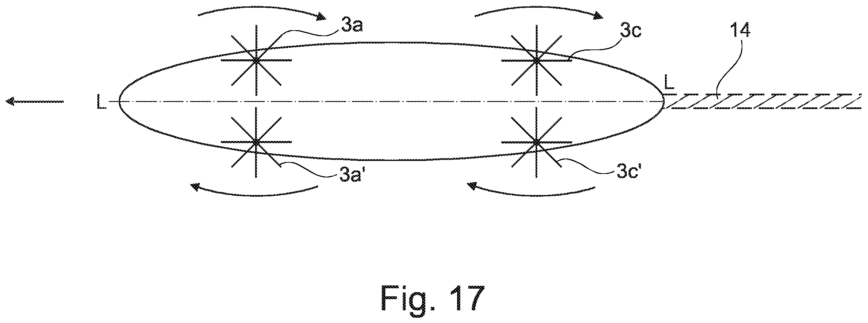

In the embodiment shown in Figure 17, the capsule body is provided with four propellers on each side. Propellers 3a, 3c are offset from propellers 3a', 3c' with respect to the longitudinal axis L of the capsule body. This arrangement may be reproduced or mirrored with propellers on the other side of the capsule body (not shown in Figure 16) and/or combined with the longitudinal offset shown in Figure 15.

It is possible to use a magnetic drive coupling so the capsule body 2 can be entirely sealed in order to protect the components therein from the colonic environment and to facilitate sterilisation.

The capsule body 2 may contain a gyroscope, compass, accelerometer and/or other sensors.

Referring to Figure 4, the capsule body 2 includes one or more connectors 13 for connection to an external power source, a control cable and/or insufflation means via a tether 14. It is envisaged that one or more of these may be provided on board so that the endoscope need not be externally tethered. The connector 13 and tether 14 may be located at one end of the capsule body 2 as shown in Figure 16. Alternatively, the connector 13 may be located on the planar underside portion of the capsule body as shown in Figure 15, with the tether 14 fitting into the channel 21.

Control may be provided either wired or wirelessly via Bluetooth or the like. An external console can be used which has a user interface (e.g. joystick, controller, display panel) such that video sent to the user interface can be viewed and recorded.

Insufflation may be provided via a rectal tube if it is not provided on board.

Power may be provided via an external power source if it is not provided on board.

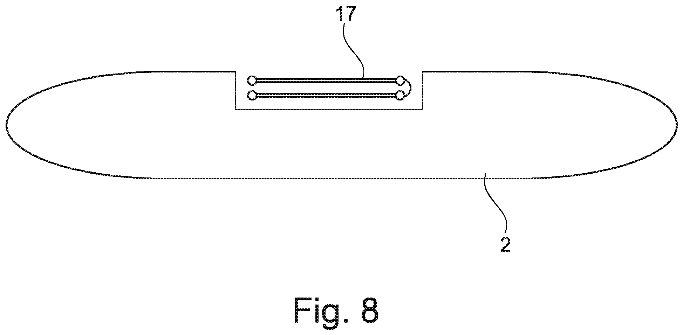

With reference to Figure 8, the endoscope may be provided with a biopsy arm 17 that is extendable from the capsule body 2. When not required, the biopsy arm is folded into a compartment of the capsule body so that the smooth external surface of the capsule body is preserved. When required, the compartment is openable to allow the biopsy arm to extend out of the compartment to obtain a sample.

The endoscope 1 is particularly useful in colonoscopy. For insertion via the rectum, the soft propellers collapse against the exterior surface of the capsule body, springing back into their operational position once inserted. The motors drive the propellers in order to rotate the blades in contact with the colon so as drive the endoscope forward, trailing the tether behind. The speed of travel is determined by the speed of rotation of the propellers and can be precisely controlled. Travel in the forward and backward directions can be achieved with all propellers (in the Figure 1 embodiment 3a, 3b, 3c, 3d) rotating in the same direction.

Alternatively, in embodiments such as that shown in Figure 16 where the propellers 3a, 3c are offset from each other with respect to the longitudinal axis L, the propellers do not rotate in the same direction. Propeller 3a is driven in a clockwise direction and propeller 3c is driven in an anticlockwise direction, in order to drive the device forwards (to the left of Figure 16). A similar arrangement is shown in Figure 17 where the uppermost propellers 3a, 3c are driven in a clockwise direction and the lowermost propellers 3a' and 3c' are driven in an anticlockwise direction. This results in forward travel in the direction indicated to the left of Figure 17.

Steering of the endoscope is achieved using the differential steering mechanism whereby the speed and direction of each of the propellers can be independently controlled in order to achieve tight turns that would not otherwise be possible.

In the above-described example, the endoscope has four propellers. It is envisaged that the invention could also be embodied by an endoscope having a different number of independently controllable propellers (but at least two, one on each side of the capsule body).

The speed and direction of rotation of each of the propellers is independently controllable using a differential steering mechanism. The differential steering mechanism means that the endoscope's direction and speed of travel can be controlled with precision and flexibility in order for the endoscope to propel itself effectively through the colon with reliable traction.

### Reference numerals

- 1: endoscope
- 2: capsule body
- 2a: capsule body base
- 2b: capsule body lid
- L: longitudinal axis of the capsule body
- 3a, 3a', 3b, 3c, 3c', 3d: propellers
- 4: blade
- 5: central hub
- 6: axle
- 7, 7a, 7b, 7c, 7d: axle channels
- 8: drive mechanism
- 9: recesses
- 10: tines
- 11: lens
- 12: light source
- 13: connector
- 14: tether
- 15: textured surface
- 16: hair-like protrusions
- 17: biopsy arm
- 18: strengthening brace
- 19: surface feature
- 20: distal end of the blade
- 21: channel in underside of capsule body
- 22: LED window
- 23: planar underside portion
- P: longitudinal plane passing through the centre of the hub

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification.

## Claims

1. A steerable endoscope comprising:
a. a capsule body having a longitudinal axis and having at least one axle channel extending transversely therein;
b. at least two propellers, each located on an axle located in said at least one axle channel, each propeller comprising a plurality of blades rotatable about a hub;
c. a camera and light source housed within said capsule body;
d. a drive mechanism, including a differential steering mechanism, housed within said capsule body, the drive mechanism being capable of driving said propellers to rotate said blades into contact with the mucosa of an internal body cavity or tract such that the endoscope is capable of self-propelled steerable travel within the internal body cavity or tract,
**characterized in that**
the blades comprise a flexible material and wherein the flexibility of the blades increases with distance from the hub.

2. The steerable endoscope of claim 1 wherein the blades comprise a resilient material.

3. The steerable endoscope of any of the preceding claims wherein the propellers further comprise a strengthening brace, for example an annular strengthening brace, attached to each blade at a point intermediate the hub and a distal end of the blade, or wherein the steerable endoscope comprises four of said propellers.

4. The steerable endoscope of any of the preceding claims wherein one or more of the blades comprises a surface feature to increase the surface area thereof, optionally wherein the surface feature comprises an enlarged distal end, bumps, ridges, protrusions, and/or hair-like protrusions.

5. The steerable endoscope of any of the preceding claims wherein the blades are symmetrical about a longitudinal plane passing through the centre of the hub, or wherein the blades are not symmetrical about a longitudinal plane passing through the centre of the hub.

6. The steerable endoscope of any of the preceding claims wherein the propellers are collapsible against the capsule body to temporarily reduce the outside dimensions of the endoscope.

7. The steerable endoscope of any of the preceding claims wherein the capsule body is sealed with respect to the or each axle, preferably further comprising ring seals on the or each axle to seal the capsule body with respect thereto, or wherein said capsule body comprises an openable and closeable two-part body, having a substantially smooth external surface when closed.

8. The steerable endoscope of any of the preceding claims further comprising a magnetic drive coupling between the drive mechanism and the or each axle, the capsule body being wholly sealed with respect to the or each axle, or further comprising one or more connectors for releasable connection of the endoscope to an external power source and/or control cable and/or insufflation means.

9. The steerable endoscope of any of the preceding claims wherein said capsule body is a spherocylinder or ovoid, or wherein said capsule body has a substantially planar underside portion.

10. The steerable endoscope of any of the preceding claims wherein said camera and light source comprises two cameras and light sources, one in each end of said capsule body.

11. The steerable endoscope of any of the preceding claims wherein the drive mechanism comprises an independently-controllable motor for each propeller.

12. The steerable endoscope of any of the preceding claims wherein said axle(s) do not protrude transversely beyond the external surface of the capsule body, or wherein one of said axle channels on one side the capsule body is longitudinally offset from another of said axle channels on the other side of the capsule body.

13. The steerable endoscope of any of the preceding claims comprising two of said axle channels, offset from one another with respect to the longitudinal axis of the capsule body.

14. The steerable endoscope of claim 12 wherein said offset axle channels are offset such that there is a clearance between one side of the outer surface of the capsule body and the distal end of the blades.

15. The steerable endoscope of any of the preceding claims further comprising means for wiping, washing or other means for clearing the lens cover of the or each camera, or claims further comprising a biopsy arm extendable from said capsule body.

## Patentansprüche

1. Lenkbares Endoskop, umfassend:
a. einen Kapselkörper mit einer Längsachse und mit mindestens einem Achsenkanal, der sich quer darin erstreckt;
b. mindestens zwei Propeller, die sich jeweils auf einer Achse befinden, die sich in dem mindestens einen Achsenkanal befindet, wobei jeder Propeller eine Vielzahl von Flügeln umfasst, die um eine Nabe drehbar sind;
c. eine Kamera und Lichtquelle, die innerhalb des Kapselkörpers untergebracht sind;
d. einen Antriebsmechanismus, der einen Differenziallenkungsmechanismus enthält, der innerhalb des Kapselkörpers untergebracht ist, wobei der Antriebsmechanismus in der Lage ist, die Propeller anzutreiben, sodass sie die Flügel in einen Kontakt mit der Schleimhaut einer inneren Körperhöhle oder eines inneren Trakts drehen, sodass das Endoskop zu einer selbstgetriebenen lenkbaren Bewegung innerhalb der inneren Körperhöhle oder des inneren Trakts in der Lage ist, **dadurch gekennzeichnet, dass**
die Flügel ein flexibles Material umfassen und wobei die Flexibilität der Flügel mit einer Entfernung von der Nabe zunimmt.

2. Lenkbares Endoskop nach Anspruch 1, wobei die Flügel ein elastisches Material umfassen.

3. Lenkbares Endoskop nach einem der vorhergehenden Ansprüche, wobei die Propeller ferner ein Verstärkungsband, zum Beispiel ein ringförmiges Verstärkungsband, umfassen, das an jedem Flügel an einem Punkt zwischen der Nabe und einem distalen Ende des Flügels angebracht ist, oder wobei das lenkbare Endoskop vier der Propeller umfasst.

4. Lenkbares Endoskop nach einem der vorhergehenden Ansprüche, wobei einer oder mehrere der Flügel ein Oberflächenmerkmal umfassen, um dessen Oberflächeninhalt zu vergrößern, wobei das Oberflächenmerkmal optional ein erweitertes distales Ende, Höcker, Rillen, Vorsprünge und/oder haarähnliche Vorsprünge umfasst.

5. Lenkbares Endoskop nach einem der vorhergehenden Ansprüche, wobei die Flügel symmetrisch zu einer Längsebene sind, die durch die Mitte der Nabe verläuft, oder wobei die Flügel nicht symmetrisch zu einer Längsebene sind, die durch die Mitte der Nabe verläuft.

6. Lenkbares Endoskop nach einem der vorhergehenden Ansprüche, wobei die Propeller gegen den Kapselkörper zusammenlegbar sind, um die Außenabmessungen des Endoskops vorübergehend zu reduzieren.

7. Lenkbares Endoskop nach einem der vorhergehenden Ansprüche, wobei der Kapselkörper in Bezug auf die oder jede Achse abgedichtet ist, bevorzugt ferner umfassend Ringdichtungen an der oder jeder Achse, um den Kapselkörper in Bezug darauf abzudichten, oder wobei der Kapselkörper einen öffenbaren und schließbaren zweiteiligen Körper umfasst, der, wenn er geschlossen ist, eine im Wesentlichen glatte Außenoberfläche aufweist.

8. Lenkbares Endoskop nach einem der vorhergehenden Ansprüche, ferner umfassend eine Magnetantriebskupplung zwischen dem Antriebsmechanismus und der oder jeder Achse, wobei der Kapselkörper in Bezug auf die oder jede Achse vollständig abgedichtet ist, oder ferner umfassend einen oder mehrere Verbinder für eine lösbare Verbindung des Endoskops mit einer externen Stromquelle und/oder einem Steuerkabel und/oder einem Insufflationsmittel.

9. Lenkbares Endoskop nach einem der vorhergehenden Ansprüche, wobei der Kapselkörper ein Sphärozylinder oder ein Ovoid ist oder wobei der Kapselkörper einen im Wesentlichen ebenen Unterseitenabschnitt aufweist.

10. Lenkbares Endoskop nach einem der vorhergehenden Ansprüche, wobei die Kamera und die Lichtquelle zwei Kameras und Lichtquellen umfasst, eine in jedem Ende des Kapselkörpers.

11. Lenkbares Endoskop nach einem der vorhergehenden Ansprüche, wobei der Antriebsmechanismus einen unabhängig steuerbaren Motor für jeden Propeller umfasst.

12. Lenkbares Endoskop nach einem der vorhergehenden Ansprüche, wobei die Achse(n) nicht quer über die Außenoberfläche des Kapselkörpers vorsteht/vorstehen oder wobei einer der Achsenkanäle auf einer Seite des Kapselkörpers in Längsrichtung von einem anderen der Achsenkanäle auf der anderen Seite des Kapselkörpers versetzt ist.

13. Lenkbares Endoskop nach einem der vorhergehenden Ansprüche, umfassend zwei der Achsenkanäle, die in Bezug auf die Längsachse des Kapselkörpers voneinander versetzt sind.

14. Lenkbares Endoskop nach Anspruch 12, wobei die versetzten Achsenkanäle derart versetzt sind, dass ein Freiraum zwischen einer Seite der Außenoberfläche des Kapselkörpers und dem distalen Ende der Flügel besteht.

15. Lenkbares Endoskop nach einem der vorhergehenden Ansprüche, ferner umfassend ein Mittel zum Wischen, Waschen oder ein anderes Mittel zum Reinigen der Linsenabdeckung der oder jeder Kamera, oder Ansprüche, ferner umfassend einen Biopsiearm, der von dem Kapselkörper ausfahrbar ist.

## Revendications

1. Endoscope orientable comprenant :
a. un corps de capsule possédant un axe longitudinal et comportant au moins un canal d'essieu s'étendant transversalement en son sein ;
b. au moins deux hélices, chacune située sur un essieu situé dans ledit au moins un canal d'essieu, chaque hélice comprenant une pluralité de pales pouvant tourner autour d'un moyeu ;
c. une caméra et une source de lumière logées à l'intérieur dudit corps de capsule ;
d. un mécanisme d'entraînement, comprenant un mécanisme d'orientation différentielle, logé à l'intérieur dudit corps de capsule, le mécanisme d'entraînement étant capable d'entraîner lesdites hélices pour faire tourner lesdites pales en contact avec la muqueuse d'une cavité ou d'un tractus interne du corps de sorte que l'endoscope soit capable de se déplacer de manière auto-propulsée et orientable à l'intérieur de la cavité ou du tractus interne du corps,
**caractérisé en ce que** les pales comprennent un matériau flexible et ladite flexibilité des pales augmentant avec la distance par rapport au moyeu.

2. Endoscope orientable de la revendication 1, lesdites pales comprenant un matériau élastique.

3. Endoscope orientable de l'une quelconque des revendications précédentes, lesdites hélices comprenant en outre une entretoise de renforcement, par exemple une entretoise de renforcement annulaire, fixée à chaque pale en un point intermédiaire entre le moyeu et une extrémité distale de la pale, ou ledit endoscope orientable comprenant quatre desdites hélices.

4. Endoscope orientable de l'une quelconque des revendications précédentes, une ou plusieurs desdites pales comprenant une caractéristique de surface pour en augmenter la superficie, éventuellement ladite caractéristique de surface comprenant une extrémité distale élargie, des bosses, des crêtes, des protubérances et/ou des protubérances similaires à des poils.

5. Endoscope orientable de l'une quelconque des revendications précédentes, lesdites pales étant symétriques par rapport à un plan longitudinal passant par le centre du moyeu, ou lesdites pales n'étant pas symétriques par rapport à un plan longitudinal passant par le centre du moyeu.

6. Endoscope orientable de l'une quelconque des revendications précédentes, lesdites hélices pouvant être repliées contre le corps de capsule de manière à réduire temporairement les dimensions extérieures de l'endoscope.

7. Endoscope orientable de l'une quelconque des revendications précédentes, ledit corps de capsule étant scellé par rapport audit ou à chaque essieu, comprenant de préférence en outre des joints annulaires sur ledit ou chaque moyeu pour sceller le corps de capsule par rapport à celui-ci, ou ledit corps de capsule comprenant un corps en deux parties pouvant être ouvert et fermé, possédant une surface externe sensiblement lisse lorsqu'il est fermé.

8. Endoscope orientable de l'une quelconque des revendications précédentes, comprenant en outre un couplage d'entraînement magnétique entre le mécanisme d'entraînement et ledit ou chaque essieu, le corps de capsule étant entièrement scellé par rapport audit ou à chaque essieu, ou comprenant en outre un ou plusieurs raccords pour un raccordement libérable de l'endoscope à une source d'alimentation externe et/ou un câble de commande et/ou un moyen d'insufflation.

9. Endoscope orientable de l'une quelconque des revendications précédentes, ledit corps de capsule étant un sphéro-cylindre ou un ovoïde, ou ledit corps de capsule possédant une partie inférieure sensiblement plane.

10. Endoscope orientable de l'une quelconque des revendications précédentes, ladite caméra et ladite source de lumière comprenant deux caméras et sources de lumière, une à chaque extrémité dudit corps de capsule.

11. Endoscope orientable de l'une quelconque des revendications précédentes, ledit mécanisme d'entraînement comprenant un moteur pouvant être commandé indépendamment pour chaque hélice.

12. Endoscope orientable de l'une quelconque des revendications précédentes, ledit ou lesdits essieux ne faisant pas saillie transversalement au-delà de la surface externe du corps de capsule, ou l'un desdits canaux d'essieu d'un côté du corps de capsule étant décalé longitudinalement par rapport à un autre desdits canaux d'essieu de l'autre côté du corps de capsule.

13. Endoscope orientable de l'une quelconque des revendications précédentes, comprenant deux desdits canaux d'essieu, décalés l'un par rapport à l'autre par rapport à l'axe longitudinal du corps de capsule.

14. Endoscope orientable de la revendication 12, lesdits canaux d'essieu décalés étant décalés de sorte qu'il existe un espace entre un côté de la surface extérieure du corps de capsule et l'extrémité distale des pales.

15. Endoscope orientable de l'une quelconque des revendications précédentes, comprenant en outre un moyen pour essuyer, laver ou un autre moyen pour nettoyer le couvercle d'objectif de ladite ou de chaque caméra, ou des revendications, comprenant en outre un bras de biopsie pouvant s'étendre depuis ledit corps de capsule.
